# EUROPÄISCHE PATENTSCHRIFT

(11) **EP 2 105 152 B1**
(45) Veröffentlichungstag und Bekanntmachung des Hinweises auf die Patenterteilung: **19.01.2011**
(21) Anmeldenummer: 09007902.1
(22) Anmeldetag: 06.09.2005
(51) Int. Cl.: A61M 1/06, F04B 45/04, F04B 49/03, F04B 49/10

(54) **Saugpumpe mit einem Sicherheitsventil**
Suction pump with safety valve
Pompe d'aspiration avec vanne de sécurité

(30) Priorität: 20.09.2004 CH 15412004
(43) Veröffentlichungstag der Anmeldung: 30.09.2009
(62) Teilanmeldung aus: 05775800.5
(73) Patentinhaber: Medela Holding AG, 6340 Baar (CH)
(72) Erfinder: Stutz, Alex, 6340 Baar (CH); Pfenniger, Erich, 6030 Ebikon (CH); Weber, Beda, 5643 Sins (CH)
(74) Vertreter: Clerc, Natalia

(56) Entgegenhaltungen:
- EP-A- 0 520 135
- WO-A-00/41745
- US-A- 1 847 658
- US-A- 4 583 970
- US-A- 4 886 494
- US-A- 6 090 065

## Beschreibung

### Technisches Gebiet

Die Erfindung betrifft eine Saugpumpe mit einem Sicherheitsventil gemäss Oberbegriff des Patentanspruchs 1.

### Stand der Technik

Saugpumpen sind für die verschiedensten Anwendungen bekannt. Sie lassen sich jedoch vorzugsweise in Brustpumpengeräten zum Abpumpen von Muttermilch oder als Drainagepumpen zum Absaugen von Körperflüssigkeiten einsetzen.

US 4,583,970 offenbart eine Handpumpe mit einem Rückschlagventil, mit welchem der Unterdruck in der Brusthaube eingestellt werden kann.

EP 0 520 135 offenbart eine Überdruckventileinrichtung für den Kühlkreis einer flüssigkeitsgekühlten Brennkraftmaschine.

US 4,886,494 und US 6,090,065 offenbaren eine Brustpumpe mit einem Entlüftungsventil.

Es gibt Saugpumpen in geschlossenen Systemen, welche im Pumpenraum stets dieselbe Luft umsetzen. Es sind jedoch auch offene Pumpsysteme mit einem Entlüftungsventil bekannt, welches sich über einen Elektromagneten zyklisch öffnen lassen.

Die Anforderungen an diese Saugpumpen, insbesondere wenn sie als Brustpumpen eingesetzt werden, sind relativ hoch. So sollten sie möglichst leistungsfähig sein und trotzdem relativ klein sein. Insbesondere in ihrer Anwendung als Brustpumpen sollten sie zudem möglichst wartungsfrei und einfach zu reinigen sein.

### Darstellung der Erfindung

Es ist deshalb eine Aufgabe der Erfindung, eine Saugpumpe mit einem Sicherheitsventil zu schaffen, welche nicht durch Flüssigkeit, insbesondere Milch, welche in die Pumpe angesaugt wurde und sich dort angesetzt hat, inaktiviert werden kann.

Diese Aufgabe löst eine Saugpumpe mit den Merkmalen des Patentanspruchs 1.

Die erfindungsgemässe Saugpumpe weist ein Sicherheitsventil mit einer ersten Stufe auf, welche bei einem ersten Unterdruck öffnet, und mit einer zweiten Stufe, welche bei einem zweiten Unterdruck öffnet. Dabei ist der erste Unterdruck betragsmässig niedriger als der zweite.

Diese zweistufige Ausbildung des Sicherheitsventils verhindert, dass Milch bis in die zweite Stufe gelangen kann. Da die erste Stufe bereits bei einer sehr geringen Abweichung des idealen Unterdrucks öffnet, wird sie im Falle eines Verklebens bei einem höherem Betrag des Unterdrucks, also im Notfall, doch noch öffnen. Das zweite Ventil verhindert, dass das Sicherheitsventil gesamthaft bei zu geringer Abweichung geöffnet wird, öffnet jedoch im Notfall zuverlässig.

Diese Pumpe eignet sich für die verschiedensten Einsatzbereiche. Insbesondere ist sie als Brustpumpe zum Absaugen von Muttermilch und als Drainagepumpe zum Absaugen von Körperflüssigkeiten geeignet. Das erfindungsgemässe Pumpenaggregat eignet sich insbesondere zur Verwendung in einer tragbaren Brustpumpe, wie sie insbesondere in der WO 2004/069306 (Anmeldenummer PCT/CH 2004/000061) beschrieben ist.

In einer bevorzugten Ausführungsform kann erfindungsgemässe Saugpumpe das Vakuum ohne allzu grossen Kraftaufwand in relativ kurzer Zeit abbauen.
Eine bevorzugte Ausführungsform und ein bevorzugtes Verfahren zur Betätigung der Saugpumpe erlauben eine möglichst kleine Ausgestaltung der Pumpe, wobei sie trotzdem kurze Pumpzyklen ermöglichen.

In diesem bevorzugten Verfahren wird ein Entlüftungskörper, welcher eine Entlüftungsöffnung des Entlüftungsventils verschliesst, zuerst nur teilweise angehoben. Dadurch ist eine kleinere Kraft notwendig als wenn die gesamte Entlüfturigsöffnung in einem einzigen Schritt freigegeben wird.

Das für die Betätigung, insbesondere die Anhebung des Entlüftungskörpers verwendete Mittel kann somit eine kleinere Leistung aufweisen und kann deshalb kleiner ausgebildet sein. Wird ein Elektromagnet als derartiges Mittel verwendet, so kann ein relativ leistungsschwaches Exemplar eingesetzt werden. Dies deshalb, weil der Elektromagnet beim Heranziehen bzw. Anheben des Entlüftungskörpers zuerst weniger Kraft aufbringen kann als am Schluss der Bewegung. Diese am Anfang zur Verfügung stehende geringere Kraft genügt jedoch, um eine kleinere Öffnung freizulegen. Es lässt sich somit ein relativ kleiner und deshalb auch kostengünstiger Elektromagnet verwenden. Zudem lässt sich die Entlüftungsöffnung relativ gross gestalten. Dadurch wird eine rasche Aufhebung des Unterdrucks und somit die gewünschte Funktionalität der Pumpe gewährleistet.

Die Pumpe erreicht ohne weiteres 120 Zyklen pro Minute. Sie lässt sich jedoch auch bei einer Zyklenzahl von 50-72 Zyklen pro Minute optimal betreiben. Die eine Zyklenzahl eignet sich besonders für die Stimulation, die andere für die Expression der Muttermilch.

In einer bevorzugten Variante des oben genannten Verfahrens wird zuerst lediglich ein Randbereich des Entlüftungskörpers von der Entlüftungsöffnung entfernt. Eine minimale Kraft ist notwendig, wenn dieser Randbereich mit einer Ecke des Entlüftungskörpers zusammenfällt.

Vorzugsweise ist der Entlüftungskörper eine Membran. Das Anheben ihres Randbereichs wird erleichtert, wenn der angehobene Randbereich eine geringere Dicke aufweist als die restliche Membran. Vorzugsweise ist die Entlüftungsöffnung vieleckig, insbesondere viereckig oder dreieckig, ausgebildet. Auch die Membran ist vorzugsweise vieleckig, vorzugsweise viereckig oder dreieckig, ausgebildet.

Zur erleichterten Anhebung der Membran kann ein Verbindungsstift an ihr befestigt oder einstückig an ihr angeformt sein, welcher mit einem Anker des Hubmagneten verbunden ist. Vorzugsweise befindet sich dieser Verbindungsstift im Randbereich der Membran, welcher die Entlüftungsöffnung bedeckt. Die Membran lässt sich jedoch auch mit einem erhöhten Flansch ausbilden, wobei der Verbindungsstift nicht über der Entlüftungsöffnung sondern an diesem erhöhten Flansch angeordnet ist.

Wenn der Verbindungsstift bezüglich der Membran bewegbar ausgebildet ist, lassen sich herstellungs- bzw. montagebedingte Toleranzen überbrücken. Auch Winkelfehler des Elektromagneten lassen sich kompensieren. Gute Resultate wurden mit einem Verbindungsstift erzielt, welcher einstückig an einer Entlüftungsmembran aus Silikon angeformt ist und welcher durch entsprechende Materialverdickung genügend Steifigkeit aufweist. Er kann gelenkig angeformt sein. In einer einfachen Ausführungsform genügt jedoch die Elastizität des Materials des Verbindungsstiftes, um ihn um seine Befestigungsstelle bewegbar auszubilden.

In einer anderen Ausführungsform weist der Entlüftungskörper einen ersten und einen zweiten Teilkörper auf. Ein erster Teilkörper verschliesst die Entlüftungsöffnung nur teilweise, da er eine Entlüftungskanal aufweist, welcher eine Verbindung von Entlüftungsöffnung nach aussen schafft. Dieser erste Teilkörper inklusive dem Entlüftungskanal ist durch einen zweiten Teilkörper verschliessbar. Beim Entlüften wird nun zuerst nur der erste Teilkörper angehoben bzw. vom Entlüftungskanal entfernt, so dass lediglich ein Teil der Entlüftungsöffnung freigegeben wird. Anschliessend lässt sich der zweite Teilkörper entfernen, so dass nun die gesamte Entlüftungsöffnung frei liegt. Vorzugsweise lässt sich in diesem zweiten Schritt der zweite Teilkörper gemeinsam mit dem ersten Teilkörper betätigen.

Die erstgenannte Ausführungsform mit der in einem Randbereich betätigbaren Entlüftungsmembran weist gegenüber dieser Ausführungsform den Vorteil auf, dass sie die Dichtheit gewährleistet und somit insbesondere bei Verwendung in einer Brustpumpe kein Leck für die abgepumpte Milch aufweisen kann.

Ein weiterer Vorteil der oben genannten Ausführungsformen ist, insbesondere bei Verwendung der Membran, dass keinerlei Federn notwendig sind und somit die Anzahl benötigter Einzelteile reduziert werden kann. Da keine Feder montiert werden muss, ist auch der Montageaufwand beim Zusammenbau der Pumpe reduziert.

Eine bevorzugte Ausführungsform hat die Aufgabe, eine Saugpumpe zu schaffen, welche möglichst einfach zusammengesetzt werden kann.

Diese Saugpumpe weist ein Entlüftungsventil mit einer Entlüftungsmembran auf, wobei diese Entlüftungsmembran und eine zur Erzeugung des Vakuums verwendete Vakuummembran einstückig in Form einer gemeinsamen Membranplatte ausgebildet sind.

Dadurch, dass Entlüftungsventil und Vakuummembran auf derselben Platte angeordnet sind, lassen sich diese Elemente kostengünstiger herstellen und einfacher montieren.

In einer bevorzugten Ausführungsform weist die Pumpe ein oberes Gehäuseteil, ein mittleres Gehäuseteil und ein unteres Gehäuseteil auf, wobei zwischen den Gehäuseteilen eine Membranplatte beziehungsweise eine Ventilplatte angeordnet ist. Durch diese Aufteilung lassen sich mehrere Elemente auf demselben Bauteil verwirklichen. Insbesondere lassen sich die für die Erzeugung des Unterdrucks notwendige Vakuummembran und die für eine schnelle Aufhebung des Unterdrucks notwendige Entlüftungsmembran einstückig auf demselben Bauteil fertigen. Auch Regelklappen bzw. Ventilklappen lassen sich einstückig auf einem gemeinsamen Bauteil fertigen. Elektromotor und Elektromagnet lassen sich in demselben Gehäuseteil befestigen. Durch Aufteilung der Pumpe in mehrere, vorzugsweise fünf Ebenen lässt sich die Anzahl Einzelteile auf ein Minimum reduzieren, ohne dass die Komplexität des Pumpaggregats aufgegeben werden muss. Dies reduziert nicht nur die äusseren Abmessungen des Pumpenaggregats, sondern minimiert auch die Herstellungs- und Montagekosten.

Ein weiterer Vorteil dieses modularen Aufbaus mit mehreren Ebene ist, dass sich die Pumpe durch einfaches Durchspülen reinigen lässt, ohne dass sie in ihre Einzelteile zerlegt werden muss. Dank des nach aussen geführten Auspuffs erübrigt sich ein Schwamm. Derartige Schwämme werden im Stand der Technik eingesetzt, um in die Pumpe gesaugte Milch aufzusaugen und auch, um den Schall zu dämpfen. Sie benötigen jedoch Platz in der Pumpe und neigen zu unerwünschter Geruchsbildung.

Dieser modulare Aufbau lässt sich auch bei einer Pumpe verwirklichen, welche nicht das oben erwähnte erfindungsgemässe Entlüftungsventil aufweist.

Weitere vorteilhafte Ausführungsformen gehen aus den abhängigen Patentansprüchen hervor.

### Kurze Beschreibung der Zeichnungen

Im folgenden wird der Erfindungsgegenstand anhand von bevorzugten Ausführungsbeispielen, welche in den beiliegenden Zeichnungen dargestellt sind, erläutert. Es zeigen:
- Figur 1: eine perspektivische Darstellung der erfindungsgemässen Saugpumpe ohne äusseres Gehäuse;
- Figur 2: eine Ansicht der Saugpumpe gemäss Figur 1 von unten;
- Figur 3: einen Längsschnitt durch die Saugpumpe gemäss Figur 1;
- Figur 4: eine Explosionsdarstellung der Saugpumpe gemäss Figur 1;
- Figur 5: eine perspektivische Darstellung eines unteren Gehäuseteils der Saug- pumpe gemäss Figur 1;
- Figur 6: eine Ansicht einer Membranplatte der Saugpumpe gemäss Figur 1 von oben;
- Figur 7: einen Längsschnitt durch die Membranplatte gemäss Figur 6;
- Figur 8: einen vergrösserten Ausschnitt der Membranplatte gemäss Figur 7;
- Figur 9: eine Ansicht in einen Teil des oberen Gehäuseteils von oben;
- Figur 10: eine Ansicht einer Membranplatte der Saugpumpe gemäss einer zweiten Ausführungsform von oben;
- Figur 11: einen Längsschnitt durch die Membranplatte gemäss Figur 10;
- Figur 12: einen vergrösserten Ausschnitt der Membranplatte gemäss Figur 11 und
- Figur 13: einen Längsschnitt durch eine erfindungsgemässe Entlüftungseinheit in einer dritten Ausführungsform.

### Wege zur Ausführung der Erfindung

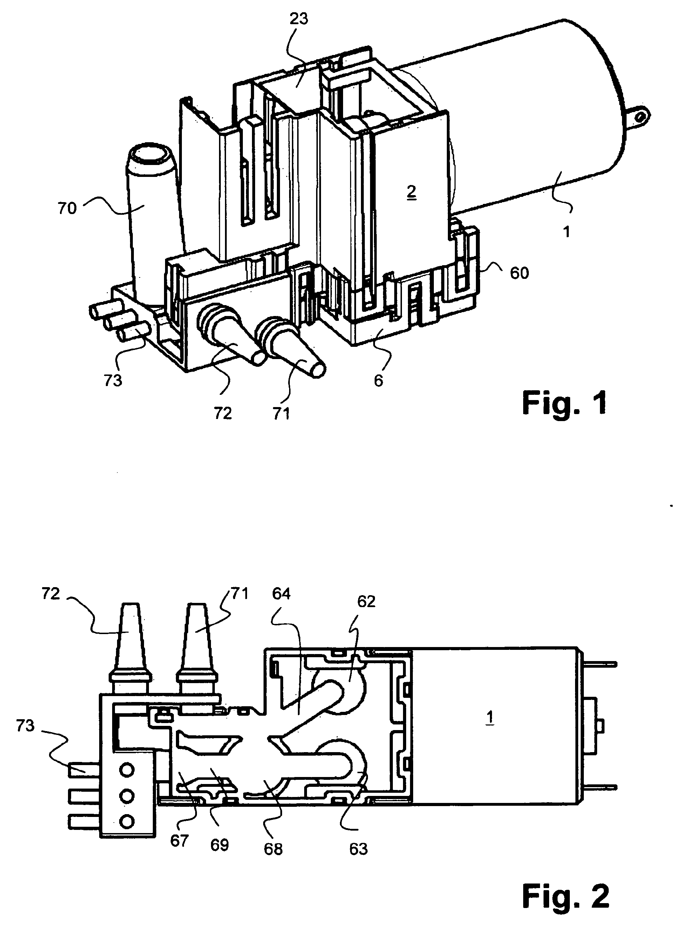

In Figur 1 ist eine erfindungsgemässe Saugpumpe dargestellt, wie sie insbesondere für ein Brustpumpengerät zum Abpumpen von menschlicher Muttermilch geeignet ist. Die Pumpe eignet sich jedoch auch für andere Anwendungen, beispielsweise für Drainagepumpen zum Absaugen von Körperflüssigkeiten.

Es ist nur das effektive Pumpenaggregat dargestellt. Dieses Aggregat ist üblicherweise in einem äusseren Gehäuse angeordnet. Dieses äussere Gehäuse und die für die Betätigung der Pumpe notwendige Elektronik und ein allfälliger Energiespeicher, beispielsweise ein Akkumulator oder eine Batterie, sind nicht dargestellt.

Die Pumpe ist äusserst kompakt aufgebaut. Eines ihrer grössten Elemente ist ein Elektromotor 1. Ferner weist sie ein oberes, ein mittleres und ein unteres Gehäuseteil 2, 4, 6 auf, welche zusammensteckbar sind. Ferner ist ein Anschlussteil 7 vorhanden, welches Bestandteil dieser Gehäuseteile ist oder, wie es hier der Fall ist, ebenfalls mit diesen zusammen gesteckt wird.

Am Anschlussteil 7 ist mindestens ein brusthaubenseitiger Anschlussstutzen 70 vorhanden, auf welcher ein Verbindungsschlauch zu einer Brusthaube aufgesteckt werden kann. Ferner verfügt das Anschlussteil 7 über einen Auspuff 71. Auch dieser Auspuff 71 führt aus dem äusseren Gehäuse heraus. Ferner ist noch ein ebenfalls aus dem äusseren Gehäuse ragender Entlüftungskanal 72 und Distanzhalter 73 vorhanden. Die Distanzhalter 73 stützen das Pumpenaggregat gegenüber einem äusseren Gehäuse ab, so dass Vibrationen nicht übertragen werden können und ein genügender Schallschutz gewährleistet ist.

In Figur 2 ist erkennbar, wie die einzelnen Anschlüsse 70, 71, 72 über einzelne Kanäle mit den einzelnen Bereichen der Pumpe verbunden sind.

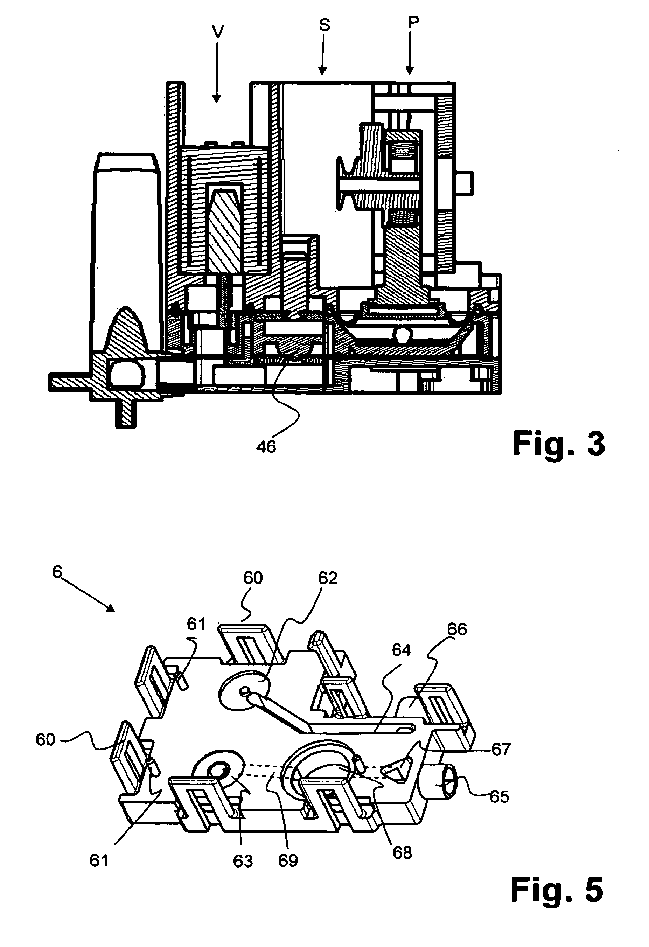

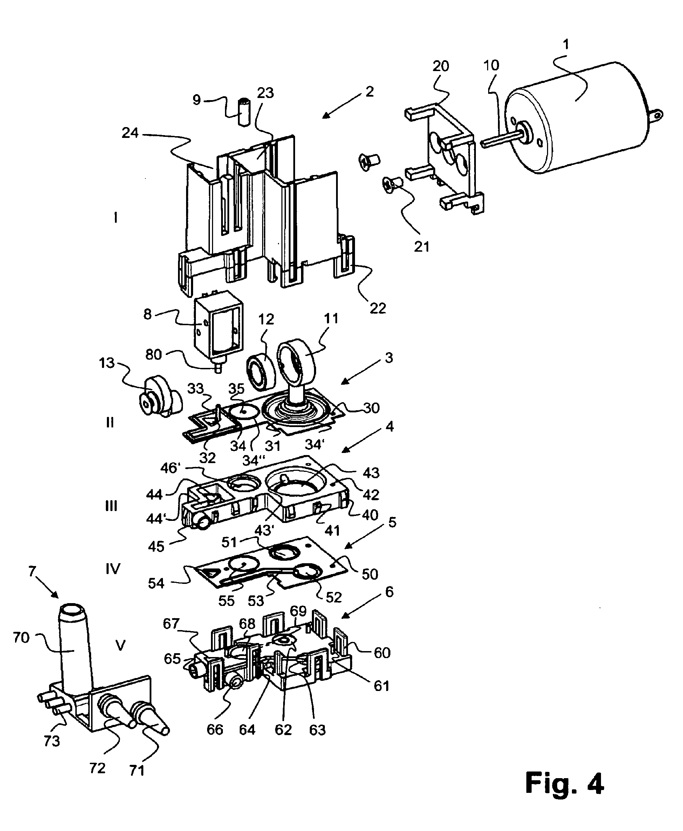

Der in Figur 3 dargestellte Längsschnitt durch die Pumpe, nun ohne Motor 1, zeigt, dass die Pumpe trotz ihrer Kompaktheit in drei klar unterscheidbare Funktionsbereiche unterteilt ist: eine Pumpeinheit P, eine Entlüftungseinheit V und eine dazwischen angeordnete Sicherheitseinheit S. Der Aufbau der Pumpe lässt sich in der Zusammenschau der Figuren 3 und 4 am besten erkennen.

Die Pumpeinheit P weist eine Vakuummembran 31 und Ein- und Auslassregelklappen 51, 52 auf, welche mit Ein- und Auslassöffnungen 62, 63 bzw. mit einer Pumpenraumöffnung 43' eine Verbindung zwischen einem Pumpenraum 43 und einem Vakuumkanal 69 schaffen. Die Vakuummembran 31 ist über eine Pleuelstange 11, Kopplungsstück 12, z.B. ein Kugellager, und Exzenter 13 mit einer Antriebswelle 10 des Elektromotors 1 verbunden und lässt sich mittels dieses Motors 1 gemäss einem vorgegebenen bzw. über eine Steuerung frei wählbaren Rhythmus bzw. Pumpkurve anheben und absenken.

Die Entlüftungseinheit V weist ein Entlüftungsventil mit einer Entlüftungsmembran 32 und eine von dieser dicht verschliessbaren Entlüftungsöffnung in Form eines Entlüftungsmembransitzes 44 auf. Die den Entlüftungsmembransitz 44 umgebene Kammer 44' ist über einen Entlüftungsanschluss 45 mit dem Entlüftungskanal 72 verbunden. Der Entlüftungsmembransitz 44 ist nach unten, d.h. auf der der Entlüftungsmembran 32 abgewandten Seite offen ausgebildet und schliesst an eine erste Entlüftungsöffnung 54 der Ventilplatte 5 und eine zweite Entlüftungsöffnung 67 des unteren Gehäuseteils 6 an. Diese zweite Entlüftungsöffnung 67 ist über den Vakuumkanal 69 mit einer Sicherheitsventilkammer 68 und der Einlassöffnung 62 verbunden.

Die Entlüftungsmembran 32 ist über einen Verbindungsstift 33 mit einem Anker 80 eines Hub- oder Elektromagneten 8 verbunden. Der Elektromagnet 8 hebt die Entlüftungsmembran 32 an und legt somit den Entlüftungsmembransitz 44 frei. Dadurch gelangt Luft über den Entlüftungskanal 72 und der ersten und zweiten Entlüftungsöffnung 54, 67 in den Vakuumkanal 69 und der darin herrschende Unterdruck wird abgebaut. Dieses Anheben und Absenken der Entlüftungsmembran 32 erfolgt ebenfalls nach einer vorgegebenen oder frei über eine Steuerung wählbaren Funktion, welche mit der Bewegung der Vakuummembran 31 koordiniert wird. Vorzugsweise werden die Bewegungen der Vakuummembran 31 und der Entlüftungsmembran 32 so koordiniert, dass eine Pumpkurve erhalten wird, wie sie in WO 01/47577 beschrieben ist und welche den Bedürfnissen von Mutter und Kind angepasst ist bzw. den natürlichen Saugrhythmus eines Säuglings imitiert. Das Aggregat funktioniert dabei in allen Lagen, dass heisst z.B. auf einem Tisch liegend bzw. stehend oder während eines Transports. Das erzeugte Vakuum ist weitgehend unabhängig davon, wie das Aggregat relativ im Raum angeordnet ist.

Die Sicherheitseinheit S weist ein Sicherheitsventil auf. Dieses Sicherheitsventil verhindert bei einer Fehlfunktion bzw. einem Ausfall der Steuerungselektronik, welche die Bewegung der Vakuummembran 31 und der Entlüftungsmembran 32 miteinander koordiniert, dass der Betrag des in der Pumpe vorherrschenden Unterdrucks zu gross wird und die Brust der Mutter verletzt wird.

Die Sicherheitseinheit S ist erfindungsgemäss zweistufig ausgebildet. Die erste Stufe besteht aus einer ersten Sicherheitsmembran 55 und einem halbkugelförmigen Sicherheitsventilverschluss 46 mit einer kleinen seitlichen Öffnung 46', welcher auf die erste Sicherheitsmembran 55 drückt. Diese erste Stufe öffnet bereits bei einem niedrigen Unterdruck von circa 120mmHg.

Die zweite Stufe weist eine zweite Sicherheitsmembran 35 auf, welche mittels einer Einstellschraube 9 verschlossen ist. Ihr Grenzwert, an welchem sie öffnet, lässt sich durch Verstellen der Einstellschraube 9 verändern. Erfindungsgemäss öffnet sie bei einem höheren Unterdruck als die erste Stufe, beispielsweise bei circa 290 mmHg.

Gelangt nun unerwünschterweise Milch oder eine andere abgesaugte Flüssigkeit in die Pumpe, so lagert sie sich im Bereich der ersten Stufe ab und kann nicht über die dazwischen liegende Kammer zur zweiten Stufe gelangen. Sie kann somit höchstens die erste Sicherheitsmembran 55 verkleben. Diese öffnet im verklebten Zustand evtl. nicht mehr bei dem voreingestellten Wert, jedoch immer noch früh genug, um eine Entlastung zu ermöglichen. Die zweite Membran öffnet immer nur dann, wenn wirklich der Grenzwert überschritten wird und die Pumpe entspannt werden muss. Da die zweite Stufe nicht verschmutzt werden kann, öffnet sie stets zuverlässig.

Die einzelnen Teile der Pumpe sind in Figur 4 am besten erkennbar. Diese Explosionsdarstellung zeigt, dass die Pumpe in mehrere Ebenen I, II, III, IV, V unterteilt ist, wobei jeweils Teile der Pumpeinheit P, der Sicherheitseinheit S und der Entlüftungseinheit V auf gemeinsamen Ebenen angeordnet sind.

Eine erste Ebene I, welche in Betriebslage üblicherweise, aber nicht zwingend, die oberste Ebene bildet, weist das obere Gehäuseteil 2, den bereits erwähnten Motor 1 sowie den Elektromagneten 8 auf. Der Motor 1 ist über Befestigungsschrauben 21 an einer Motorplatte 20 des oberen Gehäuseteils 2 befestigt. Der Motor lässt sich an die Motorplatte 20 anschrauben und ist ebenfalls an das Pumpaggregat ansteckbar. Das Gehäuseteil 2 weist eine Pleuelkammer 23 auf, welche der Aufnahme der Pleuelstange 11, des damit verbundenen Kugellagers 12 und eines Exzenters mit Gegenmasse 13 dient. Die Gegenmasse 13 muss nicht zwingend vorhanden sein. Das obere Gehäuseteil 2 weist ferner eine von der Pleuelkammer 23 getrennte Magnetkammer 24 auf, in welcher der Elektromagnet 8 befestigt ist. An ihrem unteren Ende weist das obere Gehäuseteil 2 obere Einrastbügel 22 auf, welche nach unten vorstehen.

Im oberen Gehäuseteil 2 ist ferner noch die Einstellschraube 9 für die zweite Stufe des Sicherheitsventils verstellbar angeordnet.

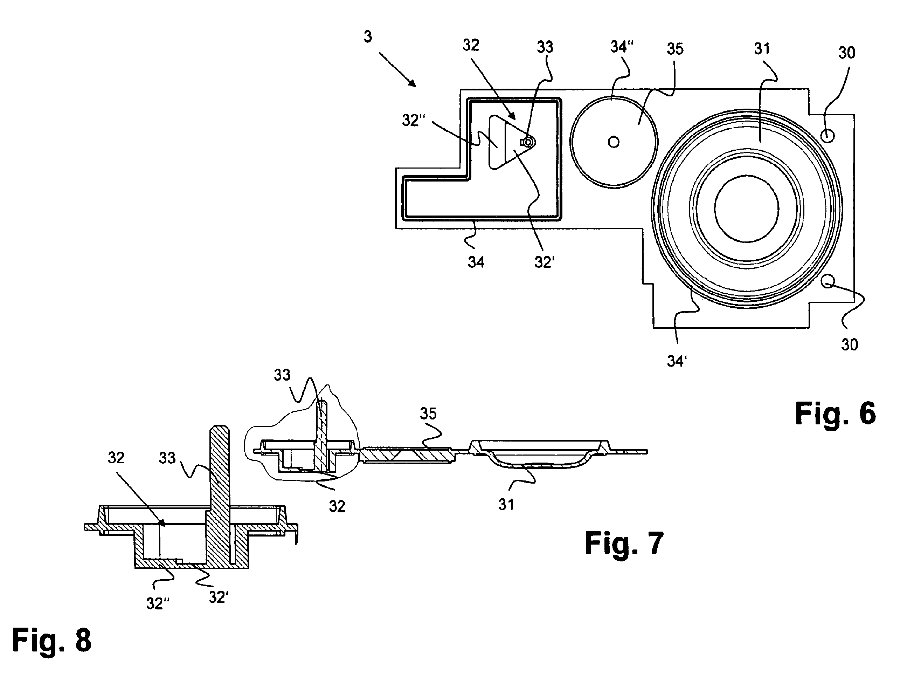

Die zweite Ebene II wird durch eine Membranplatte 3 definiert, welche sich mindestens annähernd über die gesamte Grundfläche des oberen Gehäuseteils 2 und somit des Pumpenaggregats erstreckt. Die Membranplatte 3 ist aus einem flexiblen Material, insbesondere aus Silikon, gefertigt und ist relativ dünn. Sie weist in ihren Randbereichen Zentrierlöcher 30 auf und obere bzw. untere Dichtlippen 34, 34', 34", welche eine luft- und flüssigkeitsdichte Verbindung mit dem oberen Gehäuseteil 2 bzw. mit dem mittleren Gehäuseteil 4 gewährleisten. Die unteren Dichtlippen sind in Figur 7 erkennbar. Die Membranplatte 3 umfasst die Entlüftungsmembran 32, welche dreieckförmig ausgebildet ist. Die Entlüftungsmembran 32 weist, wie dies in Figur 6 erkennbar ist, eine dreieckige Grundform auf. Dieses Dreieck ist in zwei Teilbereiche unterteilt, wobei ein erster Teilbereich 32' wiederum ein Dreieck bildet und der zweite Teilbereich 32" durch den restlichen Teil der Membran gebildet ist und somit eine Trapezform aufweist. Eine erste Ecke des ersten Teilbereichs 32' fällt mit einer Ecke der Entlüftungsmembran 32 zusammen. Eine gegenüberliegende Seite des ersten Teilbereichs 32' verläuft parallel zu einer gegenüberliegenden Seite der Entlüftungsmembran 32 und die zwei anderen Seiten des Dreiecks des ersten Teilbereichs 32' verlaufen deckungsgleich mit den Seiten des Dreiecks der Entlüftungsmembran 32. Der dreieckige erste Teilbereich 32' weist eine geringere Dicke auf als der zweite Teilbereich 32", wie dies in den Figuren 7 und 8 erkennbar ist.

An der freien Ecke des ersten Teilbereich 32' ist der Verbindungsstift 33 angeformt. Er steht, wie dies ebenfalls in den Figuren 7 und 8 erkennbar ist, mindestens annähernd senkrecht der Membranebene nach oben vor und er ist mit dem Anker 80 fest verbunden. Die Entlüftungsmembran 32 ist von einer ersten Dichtlippe 34 umgeben.

Die Membranplatte 3 umfasst ferner die Vakuummembran 31, welche mit der Pleuelstange 11 verbunden ist. Die Pleuelstange 11 kann einstückig durch Materialverdickung an der Vakuummembran 31 angeformt sein bzw. aus einem Zweikomponentenmaterial bestehen. Sie kann aber auch separat hergestellt und bei der Montage mit der Membran 31 verbunden werden. Die Vakuummembran ist mittels einer zweiten Dichtlippe 34' gegenüber dem oberen und mittleren Gehäuseteil 2, 4 abgedichtet.

Ferner weist die Membranplatte 3 noch die zweite Membran 35 des Sicherheitsventils auf. Diese weist, wie dies am besten in Figur 7 sichtbar ist, eine sich v-förmig nach unten erweiternde Öffnung auf und ist mittels einer sie umgebenden dritten Dichtlippe 34" gegenüber dem oberen und mittleren Gehäuseteil 2, 4 dichtend abgegrenzt.

Die dritte Ebene III wird durch das mittlere Gehäuseteil 4 gebildet. Dieses ist wie das obere und untere Gehäuseteil 2, 6 vorzugsweise aus einem festen Kunststoffmaterial, beispielsweise POM (Polyoxymethylen) gefertigt. Das mittlere Gehäuseteil 4 ist plattenförmig ausgestaltet und weist eine plane obere und eine plane untere Oberfläche auf. An seinen seitlichen Stirnflächen ist es mit nach oben bzw. unten ausgerichteten Rastklinken 40, 41 versehen, wobei die oberen Rastklinken 40 in die oberen Einrastbügel 22 des oberen Gehäuseteils 2 und die unteren Rastklinken 41 in untere Einrastbügel 60 des unteren Gehäuseteils 6 eingreifen. Ferner sind noch Zentrierlöcher 42 vorhanden, welche mit den Zentrierlöchern 30 der Membranplatte 3 fluchten.

Im mittleren Gehäuseteil 4 sind zwei nach unten geschlossene Ausnehmungen mit kleinen seitlichen Verbindungsöffnungen vorhanden. Eine dieser Ausnehmung bildet den Vakuummembransitz 43 für die Vakuummembran und definiert so die Pumpkammer. Eine zweite dieser Ausnehmungen bildet einen Sicherheitsverschluss 46 der ersten Sicherheitsstufe. Ferner ist im mittleren Gehäuseteil 4 die Kammer 44' mit dem darin angeordneten Membransitz 44 für die Entlüftungsmembran 32 vorhanden. Dieser Sitz 44 ist in diesem Beispiel dreieckig ausgebildet. Die Kammer 44' ist eine vertiefte, h-förmige Mulde, wobei sie Mulde mit dem Entlüftungsanschluss 45 verbunden ist.

Die vierte Ebene IV besteht wiederum aus einer flexiblen Platte, vorzugsweise aus Silikon. Sie wird durch eine Ventilplatte 5 gebildet. Auch diese weist Zentrierlöcher 50 auf, wobei mindestens ein paar dieser Zentrierlöcher 50 mit den Zentrierlöchern des mittleren Gehäuseteils 4 und der Membranplatte 3 fluchten.

Die Ventilplatte 5 weist eine erste Regelklappe 51 auf, welche einen Einlass für die Vakuumkammer bildet. Sie weist ferner eine zweite Regelklappe 52 auf, welche einen Auslass für die Vakuumkammer bildet. Von der zweiten Regelklappe 52 führt eine doppelte Dichtlippe 53 weg, welche einen im unteren Gehäuseteil 6 angeordneten, nach oben offenen Verbindungskanal 64 umgibt und diesen nach oben dichtet.

In der Ventilplatte 5 ist ferner die erste Membran 55 des Sicherheitsventils angeformt. Des weiteren ist die erste Entlüftungsöffnung 54 vorhanden, welche eine Verbindung zwischen dem Entlüftungsmembransitz 44 und einer zweiten Entlüftungsöffnung 67, welche im unteren Gehäuseteil 6 angeordnet ist, gewährleistet. Die einzelnen Elemente der Ventilplatte 5 sind wiederum mit unteren und oberen Dichtlippen versehen, um gegenüber dem mittleren und unteren Gehäuseteil 4, 6 zu dichten. Vorzugsweise sind alle Elemente der Ventilplatte 5 einstückig gemeinsam mit dieser hergestellt.

Die fünfte Ebene V umfasst das untere Gehäuseteil 6 und das Anschlussteil 7. Diese zwei Teile können durch ein einstückiges gemeinsames Teil gebildet sein oder sie können, wie es hier dargestellt ist, über Steckverbindungen miteinander gekoppelt sein.

Das untere Gehäuseteil 6 ist in Figur 4 und in einer anderen Perspektive in Figur 5 dargestellt. Es ist ebenfalls plattenförmig ausgebildet, wobei seine Unterseite den Boden des Pumpenaggregats bildet. Seitlich stehen die unteren Einrastbügel 60 nach oben vor, so dass die unteren Einrastklinken 41 des mittleren Gehäuseteils 4 in diese einrasten können. Ferner sind Zentrierstifte 61 vorhanden, welche ebenfalls nach oben vorstehen und welche durch die Zentrierlöcher 30, 42, 50 der Membranplatte 3, des mittleren Gehäuseteils 4 und der Ventilplatte 5 geführt werden können. Diese Zentrierstifte und Zentrierlöcher erleichtern das Aufeinanderstapeln der einzelnen Ebenen und ermöglichen somit eine schnelle Montage.

Das untere Gehäuseteil 6 weist ferner eine erste kreisförmige Ausnehmung mit zentraler Erhöhung auf, welche eine Einlassöffnung 62 bildet. Eine zweite kreisförmige Ausnehmung bildet eine Auslassöffnung 63. Diese Einlassöffnung 62 ist mit dem im inneren des unteren Gehäuseteils 6 verlaufenden Vakuumkanal 69 verbunden. Dieser Kanal 69 durchdringt zuerst die Sicherheitsventilöffnung 68, welche ebenfalls als Mulde ausgebildet ist und über welche die Sicherheitsmembran 55 der ersten Stufe angeordnet ist.

Der Verbindungskanal 64, welcher oben offen und über die Dichtlippe 53 gedichtet im unteren Gehäuseteil 6 verläuft, mündet die Auslassöffnung 63. Dieser Verbindungskanal 64 ist mit der im unteren Gehäuseteil 6 vorhanden zweiten Entlüftungsöffnung 67 verbunden.
Wie in Figur 5 erkennbar ist, endet der Verbindungskanal 64 in einem Auspuffanschluss 66, welcher in den Auspuff 71 einsteckbar ist. Die zweite Entlüftungsöffnung 67 führt zu einem brusthaubenseitigen Anschlussteil 65, welcher mit dem brusthaubenseitigen Anschlussstutzen 70 verbunden ist.

Dieser Aufbau ermöglicht ein einfaches Reinigen der Pumpe. Sollte Milch oder eine andere abgesaugt Flüssigkeit in die Pumpe gelangt sein, so lässt sich diese einfach mit Wasser oder Luft durchspülen, indem das Reinigungsmedium durch den brustpumpenseitigen Anschluss 70 hineingepresst oder geblasen wird, wobei dieses die Pumpe durch den Entlüftungskanal 72 und dem Auspuff 71 wieder verlässt.

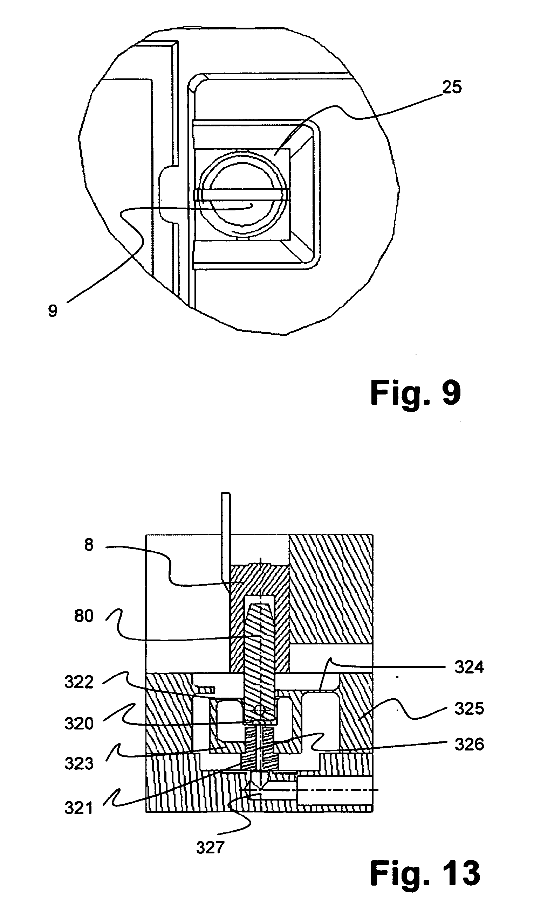

In Figur 9 ist eine Teilansicht von oben in den oberen Gehäuseteil 2 dargestellt. Wie hier erkennbar ist, ist die Einstellschraube 9 des Sicherheitsventils S mit einem runden Querschnitt ausgebildet, aber in einer viereckigen Gewindeöffnung 25 eingeschraubt. Dadurch ist stets ein genügend grosser Luftdurchlass gewährleistet.

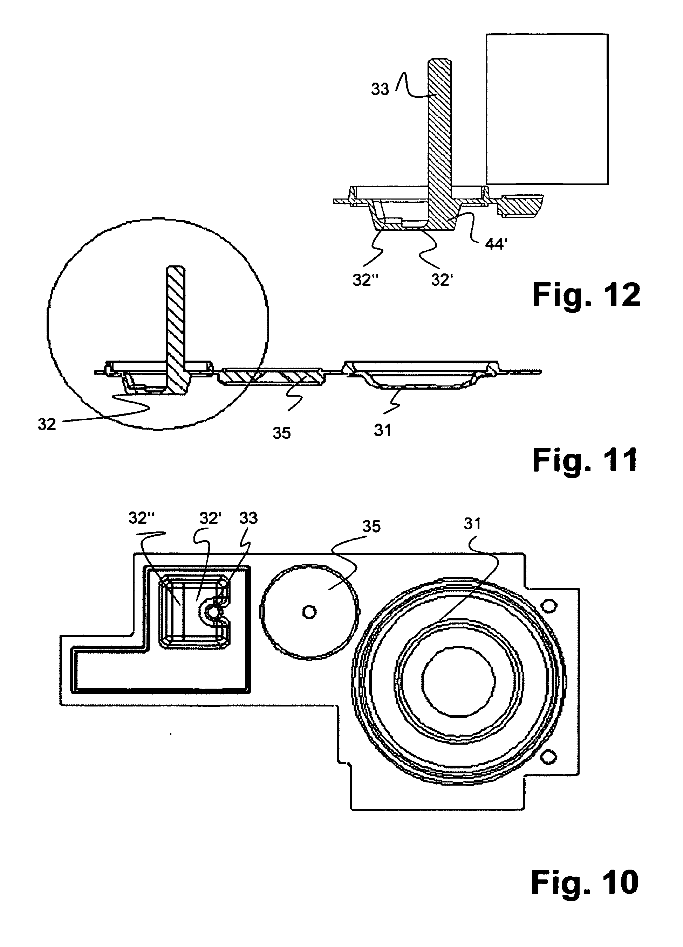

In den Figuren 10 bis 12 ist eine zweite Ausführungsform dargestellt. Diese Ausfuhrungsform unterscheidet sich von der oben beschriebenen im Bereich des Entlüftungsventils. Die übrigen Teile sind gleich ausgebildet und werden hier deshalb nicht nochmals dargestellt und beschrieben. In diesem Beispiel ist die Entlüftungsmembran 32 nicht dreieckig sondern viereckig ausgebildet. Sie kann jedoch auch sechsoder achteckig bzw. eine beliebige vieleckige Form aufweisen. Es hat sich jedoch in der Praxis gezeigt, dass die viereckige Variante im Vergleich zur dreieckigen Form zuverlässiger schliesst und öffnet. Wiederum ist die Entlüftungsmembran in einen dicken und einen dünnen Teilbereich 32', 32" unterteilt, wobei der Verbindungsstift 33 am dünnen Teilbereich 32' angeordnet ist. Die zwei Teilbereiche 32', 32" sind ebenfalls im wesentlichen viereckig ausgebildet, wobei der Verbindungsstift 33 an einer dem dicken Teilbereich 32" abgewandten Längsseite des dünnen Teilbereichs 32' angeordnet ist. Er ist nun nicht mehr in einer Ecke befestigt, sondern ungefähr in der Mitte dieser Längsseite, wie dies in Figur 10 erkennbar ist. Es hat sich auch gezeigt, dass die Funktionalität besser gewährleistet ist, wenn der Verbindungsstift an der Seitenwand 44" der Kammer 44' angeformt ist, wie dies in Figur 12 dargestellt ist. Die darunterliegende Öffnung, dass heisst der Entlüftungsmembransitz 44, ist vorzugsweise jedoch nach wie vor dreieckig ausgebildet. In diesem Fall ist der Verbindungsstift 33 vorzugsweise genau über der Ecke des dreieckigen Lochs 44 angeordnet.

In Figur 13 ist eine weitere Ausführungsform des erfindungsgemässen Entlüftungsventils dargestellt. Die restliche Pumpe kann gleich ausgebildet sein wie oben beschrieben und sie wird hier deshalb nicht nochmals dargestellt. Der Entlüftungskörper ist hier nicht eine Membran, sondern er ist durch einen ersten und zweiten Entlüftungsblock 320, 321 gebildet.

Der erste Entlüftungsblock 320 ist mit dem Anker 80 des Hubmagneten 8 fest verbunden. Er ist über ein erstes, weiches Federelement 322 und einen Haltebügel 323 mit dem zweiten Entlüftungsblock 321 verbunden. Der Haltebügel 323 ist über ein zweites, härteres Federelement 324 an einem den Entlüftungskörper umgebenden Basiskörper 325 befestigt. Basiskörper 325, erster Entlüftungsblock 320, Haltebügel 323 sowie die zwei Federelemente 322, 324 sind vorzugsweise aus Kunststoff gefertigt und alle gemeinsam einstückig ausgebildet.

Der zweite Entlüftungsblock 321 weist einen ersten Entlüftungskanal 326 auf, welcher vom ersten Entlüftungsblock 320 verschliessbar ist. Der zweite Entlüftungsblock 321 verschliesst einen zweiten Entlüftungskanal 327, welcher mit dem Vakuumkanal 69 verbunden ist. Der zweite Entlüftungskanal 327 weist einen grösseren Durchmesser auf als der erste Entlüftungskanal 326.

Wird nun der Anker 80 des Magneten 8 angehoben, so wird zuerst aufgrund des weichen Federelements 322 nur der erste Entlüftungsblock 320 angehoben und der erste Entlüftungskanal 326 wird freigegeben. Wird der Anker 80 weiter angehoben, so ist der Magnet nun leistungsfähiger und er kann nun die trotz des härteren Federelements 324 den zweiten Entlüftungsblock 321 anheben und somit den zweiten Entlüftungskanal 327 freigeben. Dadurch ist nun eine Verbindung zwischen erstem Entlüftungskanal 326 und Vakuumkanal 69 geschaffen und der Unterdruck in der Pumpe wird abgebaut. Alternativ bzw. ergänzend zu den unterschiedlich starken Federn können Anschläge vorhanden sein, welche den Weg der ersten und zweiten Feder begrenzen.

Auch bei dieser Ausführungsform wird somit der Entlüftungskörper so angehoben, dass er zuerst nur einen Teil der Entlüftungsöffnung freigibt, so dass zu Beginn der Anhebung mit einer kleineren Kraft gearbeitet werden kann als am Schluss der Bewegung.

Die erfindungsgemässe Saugpumpe weist somit mehrere Vorteile auf. Sie bietet auf kleinstem Raum eine grosse Funktionalität und ist zudem kostengünstig herstellbar und einfach montierbar.

### Bezugszeichenliste

- P: Pumpeinheit
- V: Entlüftungseinheit
- S: Sicherheitseinheit

- I: erste Ebene
- II: zweite Ebene
- III: dritte Ebene
- IV: vierte Ebene
- V: fünfte Ebene

- 1: Motor
- 10: Antriebswelle
- 11: Pleuelstange
- 12: Kugellager
- 13: Exzenter mit Gegenmasse

- 2: oberes Gehäuseteil
- 20: Motorplatte
- 21: Befestigungsschrauben
- 22: Obere Einrastbügel
- 23: Pleuelkammer
- 24: Magnetkammer
- 25: Gewindeöffnung

- 3: Membranplatte
- 30: Zentrierloch
- 31: Vakuummembran
- 32: Entlüftungsmembran
- 32': erster Teilbereich
- 32": zweiter Teilbereich

- 33: Verbindungsstift
- 34: erste Dichtlippe
- 34': zweite Dichtlippe
- 34": dritte Dichtlippe
- 35: Zweite Membran des Sicherheitsventils
- 320: erster Entlüftungsblock
- 321: zweiter Entlüftungsblock
- 322: erstes Federelement
- 323: Haltebügel
- 324: zweites Federelement
- 325: Basiskörper
- 326: Erster Entlüftungskanal
- 327: Zweiter Entlüftungskanal

- 4: Mittleres Gehäuseteil
- 40: obere Rastklinken
- 41: untere Rastklinken
- 42: Zentrierloch
- 43: Vakuummembransitz
- 43': Pumpenraumöffnung
- 44: Entlüftungsmembransitz
- 44': Kammer
- 44": Seitenwand
- 45: Entlüftungsanschluss
- 46: Sicherheitsventilverschluss der ersten Stufe
- 46': seitliche Öffnung

- 5: Ventilplatte
- 50: Zentrierloch
- 51: Erste Regelklappe
- 52: Zweite Regelklappe
- 53: Dichtlippe für Verbindungskanal
- 54: Erste Entlüftungsöffnung
- 55: Erste Membran des Sicherheitsventils

- 6: Unteres Gehäuseteil
- 60: untere Einrastbügel
- 61: Verbindungsstift
- 62: Einlassöffnung
- 63: Auslassöffnung
- 64: Verbindungskanal
- 65: Brusthaubenseitiges Anschlussteil
- 66: Auspuffanschluss
- 67: Zweite Entlüftungsöffnung
- 68: Sicherheitsventilkammer
- 69: Vakuumkanal

- 7: Anschlussteil
- 70: brustpumpenseitiger Anschlussstutzen
- 71: Auspuff
- 72: Entlüftungskanal
- 73: Distanzhalter

- 8: Hubmagnet
- 80: Anker

- 9: Einstellschraube

## Patentansprüche

1. Saugpumpe mit einem Sicherheitsventil, wobei das Sicherheitsventil eine erste Stufe (55, 46) aufweist, welche bei einem ersten Unterdruck öffnet, **dadurch gekennzeichnet, dass** das Sicherheitsventil eine zweite Stufe (35, 9) aufweist, welche bei einem zweiten Unterdruck öffnet, wobei der erste Unterdruck betragsmässig niedriger ist als der zweite.

2. Saugpumpe nach Anspruch 1, wobei ein Entlüftungsventil eine Entlüftungsmembran (32) aufweist und wobei diese Entlüftungsmembran (32) und eine zur Erzeugung des Vakuums verwendete Vakuummembran (31) einstückig in Form einer gemeinsamen Membranplatte (3) ausgebildet sind.

3. Saugpumpe nach Anspruch 2, wobei ein Teil dieser Membranplatte (3) als Sicherheitsmembran (35) des Sicherheitsventils ausgebildet ist.

4. Saugpumpe nach einem der Ansprüche 2 oder 3, wobei die Pumpe ein oberes Gehäuseteil (2) zur Aufnahme eines Hubmagneten (8) zur Betätigung der Entlüftungsmembran (32) und zur Aufnahme eines Elektromotors (1) zur Betätigung der Vakuummembran (31), ein mittleres Gehäuseteil (4) mit einem eine Entlüftungsöffnung umgebender Entlüftungsmembransitz (44) für die Entlüftungsmembran (32) und mit einem Vakuummembransitz (43) für die Vakuummembran (31) und ein unteres Gehäuseteil (6) zur Ausbildung eines Vakuumkanals (69) zwischen Entlüftungsöffnung (44, 54, 67) und einer Einlassöffnung (62, 51, 43') des Vakuumnembransitzes (43) aufweist, wobei zwischen dem oberen und mittleren Gehäuseteil (2, 4) die Membranplatte (3) und zwischen dem mittleren und unteren Gehäuseteil (4, 6) eine Ventilplatte (5) angeordnet ist.

5. Saugpumpe nach Anspruch 4, wobei die Ventilplatte (5) und die Membranplatte (3) im wesentlichen plan ausgebildet sind.

6. Saugpumpe nach einem der Ansprüche 4 oder 5, wobei die Ventilplatte (5) und die Membranplatte (3) aus einem Kunststoff, insbesondere aus Silikon, gefertigt sind.

7. Saugpumpe nach einem der Ansprüche 2 bis 6, wobei sie einen Auspuff (71) und wahlweise einen Vakuumanschluss (70) und einen Entlüftungsanschluss (72) aufweist, welche aus einem äusseren Gehäuse der Pumpe ragen.

8. Saugpumpe nach den Ansprüchen 4 und 7, wobei am unteren Gehäuseteil (6) ein Auspuffanschluss (66) angeformt ist, welcher mit dem an einem Anschlussteil (7) angeformten Auspuff (71) verbindbar ist.

9. Saugpumpe nach einem der Ansprüche 4 bis 8, wobei das untere, das mittlere und das obere Gehäuseteile (2, 4, 6) steckbar zwecks Bildung eines geschlossenen Aggregatgehäuses miteinander verbindbar sind.

10. Saugpumpe nach Anspruch 9, wobei das Aggregatgehäuse im wesentlichen wasserdicht ausgebildet ist und durch einen brustpumpenseitige Anschluss (70), dem Auspuff (71) und der Entlüftungsöffnung (72) durchspülbar ist.

11. Saugpumpe nach einem der Ansprüche 1 bis 10, wobei die Saugpumpe ein Entlüftungsventil aufweist, wobei das Entlüftungsventil eine Entlüftungsöffnung (44, 54, 67), einen die Entlüftungsöffriung (44, 54, 67) dichtend verschliessbaren Entlüftungskörper (32) und ein Betätigungsmittel (8) zur Betätigung des Entlüftungskörpers (32) aufweist, **dadurch gekennzeichnet, dass** der Entlüftungskörper (32) derart betätigbar ist, dass er beim Öffnen des Ventils zuerst nur einen Teilbereich der Entlüftungsöffnung (44, 54, 67) freigibt und anschliessend einen grösseren Teilbereich oder die gesamte EntlüßungsöHnung (44, 54, 67) freigibt.

12. Saugpumpe nach Anspruch 11, wobei der Entlüftungskörper eine Entlüftungsmembran (32) ist und wobei die Entlüftungsmembran (32) einen Randbereich (32') aufweist, an welchem sie beim Öffnen des Ventils zuerst von der Entlüftungsöffnung (44, 54, 67) entfernbar ist.

13. Saugpumpe nach Anspruch 12, wobei das Betätigungsmittel (8) ein Hubmagnet ist, wobei die Entlüftungsmembran (32) über einen Verbindungsstift (33) mit einem Anker (11) des Hubmagneten (8) verbunden ist und wobei der Verbindungsstift (33) an diesem Randbereich der Entlüftungsmembran (32) mit dieser verbunden ist.

14. Saugpumpe nach einem der Ansprüche 12 bis 13, wobei die Entlüftungsmembran (32) einen ersten Teilbereich (32') mit einer geringeren Dicke und einen zweiten Teilbereich (32") mit einer grösseren Dicke aufweist und wobei der Randbereich im ersten Teilbereich (32') angeordnet ist.

15. Saugpumpe nach Anspruch 11, wobei der Entlüftungskörper einen ersten und einen zweiten Teilkörper (320, 321) aufweist, wobei der erste Teilkörper (320) mit einem Anker (80) eines Hubmagneten (8) verbunden ist und der zweite Teilkörper (321) über ein erstes Federelement (322) mit dem ersten Teilkörper (320) verbunden ist und wobei der zweite Teilkörper (321) einen ersten Entlüftungskanal (326) aufweist, welcher vom ersten Teilkörper (320) verschließbar ist, wobei der zweite Teilkörper (321) einen zweiten Entlüftungskanal (327) verschliesst und wobei der zweite Entlüftungskanal (327) einen grösseren Durchmesser aufweist als der erste Entlüftungskanal (326).

## Claims

1. Suction pump with a safety valve, in which the safety valve has a first step (55, 46) which opens at a first underpressure, **characterized in that** the safety valve has a second step (35, 9) which opens at a second underpressure, the first underpressure being quantitatively lower than the second.

2. Suction pump according to Claim 1, in which an air release valve has an air release membrane (32), and in which this air release membrane (32) and a vacuum membrane (31) used to generate the vacuum are designed integrally in the form of a common membrane plate (3).

3. Suction pump according to Claim 2, in which a part of this membrane plate (3) is designed as a safety membrane (35) of a safety valve.

4. Suction pump according to either of Claims 2 and 3, in which the pump comprises an upper housing part (2) which receives a lifting magnet (8) for actuating the air release membrane (32) and receives an electric motor (1) for actuating the vacuum membrane (31), a middle housing part (4) with an air release membrane seat (44) which is provided for the air release membrane (32) and which surrounds an air release opening, and with a vacuum membrane seat (43) for the vacuum membrane (31), and a lower housing part (6) for forming a vacuum channel (69) between air release opening (44, 54, 67) and an inlet opening (62, 51, 43') of the vacuum membrane seat (43), the membrane plate (3) being arranged between the upper and middle housing parts (2, 4), and a valve plate (5) being arranged between the middle and lower housing parts (4, 6).

5. Suction pump according to Claim 4, in which the valve plate (5) and the membrane plate (3) are substantially plane.

6. Suction pump according to either of Claims 4 and 5, in which the valve plate (5) and the membrane plate (3) are made of a plastic, in particular of silicone.

7. Suction pump according to one of Claims 2 to 6, comprising an exhaust (71) and, optionally, a vacuum connecting element (70) and an air release connecting element (72) which protrude from an outer housing of the pump.

8. Suction pump according to Claims 4 and 7, in which an exhaust connecting element (66) is formed integrally on the lower housing part (6) and can be connected to the exhaust (71) formed integrally on a connecting part (7).

9. Suction pump according to one of Claims 4 to 8, in which the lower, middle and upper housing parts (2, 4, 6) can be plugged together so as to form a closed housing assembly.

10. Suction pump according to Claim 9, in which the housing assembly is substantially watertight and can be flushed through via a breast pump connecting element (70) to the exhaust (71) and the air release opening (72).

11. Suction pump according to one of Claims 1 to 10, the suction pump having an air release valve, the air release valve having an air release opening (44, 54, 67), an air release body (32) which can close the air release opening (44, 54, 67) sealingly, and an actuating means (8) for actuating the air release body (32), **characterized in that** the air release body (32) can be actuated in such a way that, upon opening of the valve, it at first frees only a partial area of the air release opening (44, 54, 67) and thereafter frees a larger partial area or the entire air release opening (44, 54, 67).

12. Suction pump according to Claim 11, in which the air release body is an air release membrane (32), and in which the air release membrane (32) has an edge area (32') at which it can at first be removed from the air release opening (44, 54, 67) upon opening of the valve.

13. Suction pump according to Claim 12, in which the actuating means (8) is a lifting magnet, and the air release membrane (32) is connected to an armature (11) of the lifting magnet (8) via a connecting pin (33), and the connecting pin (33) is connected to the air release membrane (32) at this edge area thereof.

14. Suction pump according to either of Claims 12 and 13, in which the air release membrane (32) has a first partial area (32') with a lesser thickness and a second partial area (32") with a greater thickness, and in which the edge area is arranged in the first partial area (32').

15. Suction pump according to Claim 11, in which the air release body has first and second subsidiary bodies (320, 321), the first subsidiary body (320) being connected to an armature (80) of a lifting magnet (8), and the second subsidiary body (321) being connected to the first subsidiary body (320) via a first spring element (322), and the second subsidiary body (321) having a first air release channel (326) which can be closed by the first subsidiary body (320), the second subsidiary body (321) closing a second air release channel (327), and the second air release channel (327) having a greater diameter than the first air release channel (326).

## Revendications

1. Pompe de succion, comprenant une soupape de sécurité, dans laquelle la soupape de sécurité présente un premier étage (55, 46), qui s'ouvre sous l'effet d'une première dépression, **caractérisée en ce que** la soupape de sécurité présente un deuxième étage (35, 9), qui s'ouvre sous l'effet d'une deuxième dépression, la première dépression étant considérablement inférieure à la deuxième dépression.

2. Pompe de succion selon la revendication 1, dans laquelle une soupape de ventilation présente une membrane de ventilation (32) et dans laquelle cette membrane de ventilation (32) et une membrane de vide (31) utilisée pour produire du vide sont réalisées d'une seule pièce sous forme de plaque à membrane commune (3).

3. Pompe de succion selon la revendication 2, dans laquelle une partie de cette plaque à membrane (3) est réalisée sous forme de membrane de sécurité (35) de la soupape de sécurité.

4. Pompe de succion selon l'une quelconque des revendications 2 ou 3, dans laquelle la pompe présente une partie de boîtier supérieure (2) pour recevoir un aimant de levage (8) pour l'actionnement de la membrane de ventilation (32) et pour recevoir un moteur électrique (1) pour l'actionnement de la membrane de vide (31), une partie de boîtier centrale (4) avec un siège de membrane de ventilation (44) entourant une ouverture de ventilation, pour la membrane de ventilation (32) et avec un siège de membrane de vide (43) pour la membrane de vide (31), et une partie de boîtier inférieure (6) pour créer un canal de vide (69) entre l'ouverture de ventilation (44, 54, 67) et une ouverture d'entrée (62, 51, 43') du siège de la membrane de vide (43), la plaque à membrane (3) étant disposée entre la partie de boîtier supérieure (2) et la partie de boîtier centrale (4), et une plaque de soupape (5) étant disposée entre la partie de boîtier centrale (4) et la partie de boîtier inférieure (6).

5. Pompe de succion selon la revendication 4, dans laquelle la plaque de soupape (5) et la plaque de membrane (3) sont réalisées sous forme essentiellement plane.

6. Pompe de succion selon l'une quelconque des revendications 4 ou 5, dans laquelle la plaque de soupape (5) et la plaque à membrane (3) sont fabriquées en plastique, notamment en silicone.

7. Pompe de succion selon l'une quelconque des revendications 2 à 6, présentant un échappement (71) et, au choix, une connexion à vide (70) et une connexion de ventilation (72) qui font saillie hors d'un boîtier extérieur de la pompe.

8. Pompe de succion selon les revendications 4 et 7, dans laquelle une connexion d'échappement (66) est moulée au niveau de la partie de boîtier inférieure (6), laquelle peut être connectée à l'échappement (71) moulé sur une partie de connexion (7).

9. Pompe de succion selon l'une quelconque des revendications 4 à 8, dans laquelle la partie de boîtier inférieure (6), la partie de boîtier centrale (4) et la partie de boîtier supérieure (2) peuvent être connectées les unes aux autres par emboîtement dans le but de former un boîtier unitaire fermé.

10. Pompe de succion selon la revendication 9, dans laquelle le boîtier unitaire est réalisé sous forme essentiellement étanche à l'eau et peut être purgé par une connexion du côté du tire-lait (70), par l'échappement (71) et par l'ouverture de ventilation (72).

11. Pompe de succion selon l'une quelconque des revendications 1 à 10, la pompe de succion présentant une soupape de ventilation, la soupape de ventilation présentant une ouverture de ventilation (44 ,54, 67), un corps de ventilation (32) pouvant fermer hermétiquement l'ouverture de ventilation (44, 54, 67) et un moyen d'actionnement (8) pour l'actionnement du corps de ventilation (32), **caractérisée en ce que** le corps de ventilation (32) peut être actionné de telle sorte qu'il libère, lors de l'ouverture de la soupape, d'abord seulement une première région partielle de l'ouverture de ventilation (44, 54, 67) et qu'il libère ensuite une région partielle plus grande ou toute l'ouverture de ventilation (44, 54, 67).

12. Pompe de succion selon la revendication 11, dans laquelle le corps de ventilation est une membrane de ventilation (32) et dans laquelle la membrane de ventilation (32) présente une région de bord (32') au niveau de laquelle elle peut être enlevé d'abord de l'ouverture de ventilation (44, 54, 67) lors de l'ouverture de la soupape.

13. Pompe de succion selon la revendication 12, dans laquelle le moyen d'actionnement (8) est un aimant de levage, la membrane de ventilation (32) étant connectée par le biais d'une goupille de connexion (33) à une armature (11) de l'aimant de levage (8) et la goupille de connexion (33) étant connectée à la membrane de ventilation (32) au niveau de sa région de bord.

14. Pompe de succion selon l'une quelconque des revendications 12 ou 13, dans laquelle la membrane de ventilation (32) présente une première région partielle (32') avec une plus faible épaisseur et une deuxième région partielle (32") avec une plus grosse épaisseur et dans laquelle la région de bord est disposée dans la première région partielle (32').

15. Pompe de succion selon la revendication 11, dans laquelle le corps de ventilation présente un premier et un deuxième corps partiels (320, 321), le premier corps partiel (320) étant connecté à une armature (80) d'un aimant de levage (8) et le deuxième corps partiel (321) étant connecté par le biais d'un premier élément de ressort (322) au premier corps partiel (320), et le deuxième corps partiel (321) présentant un premier canal de ventilation (326), qui peut être fermé par le premier corps partiel (320), le deuxième corps partiel (321) fermant un deuxième canal de ventilation (327) et le deuxième canal de ventilation (327) présentant un plus grand diamètre que le premier canal de ventilation (326).
